(19) Europäisches Patentamt
European Patent Office
Office européen des brevets

(11) **EP 4 079 358 B1**

(12) **EUROPÄISCHE PATENTSCHRIFT**

(45) Veröffentlichungstag und Bekanntmachung des Hinweises auf die Patenterteilung:
**19.11.2025 Patentblatt 2025/47**

(21) Anmeldenummer: **22167685.1**

(22) Anmeldetag: **11.04.2022**

(51) Internationale Patentklassifikation (IPC):
***A61M 16/00*** *(2006.01)*

(52) Gemeinsame Patentklassifikation (CPC):
**A61M 16/024;** A61M 2016/0027; A61M 2016/0036;
A61M 2205/3334; A61M 2205/3341;
A61M 2205/3561; A61M 2205/502; A61M 2230/42

(54) **BEATMUNGSGERÄT ZUR MASCHINELLEN BEATMUNG EINES PATIENTEN**

VENTILATION APPARATUS FOR MECHANICAL VENTILATION OF A PATIENT

RESPIRATEUR PERMETTANT DE FAIRE RESPIRER ARTIFICIELLEMENT UN PATIENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.04.2021 DE 102021110429**

(43) Veröffentlichungstag der Anmeldung:
**26.10.2022 Patentblatt 2022/43**

(73) Patentinhaber: **Drägerwerk AG & Co. KGaA 23558 Lübeck (DE)**

(72) Erfinder:
• **Bender, Michael 23558 Lübeck (DE)**
• **von Blumenthal, Tilman 23558 Lübeck (DE)**

(56) Entgegenhaltungen:
EP-A1- 1 961 378     EP-A1- 3 698 713
EP-A1- 3 769 668     EP-A2- 0 127 905
WO-A1-2016/067619     DE-T2- 69 623 400

EP 4 079 358 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft ein Beatmungsgerät zur maschinellen Beatmung eines Patienten.

Beatmungsgeräte mit voreingestellten Beatmungsdruck-Verläufen zur maschinellen Beatmung von Patienten sind bekannt. Dabei können abhängig von dem Zustand des Patienten Parameter der Beatmung eingestellt werden, wie etwa der positive endexspiratorische Druck (PEEP), der mittlere Beatmungsdruck, das Tidalvolumen, die Atemfrequenz und dergleichen.

Um eine Beatmung automatisiert oder teilautomatisiert durchzuführen, muss bekanntermaßen über mindestens einen Sensor ein Gasfluss und/oder ein Gasfluss-abhängiger Messwert in regelmäßigen zeitlichen Abständen gemessen werden. Dadurch kann der Gasfluss während einer Inspirationszeit und während einer Exspirationszeit des entsprechenden Atemzyklus überwacht werden.

Aus WO 2016 / 067 619 A1 ist ein Beatmungsgerät bekannt. Das Beatmungsgerät ist eingerichtet, das Auftreten eins dPEEP aus einem gemessenen exspiratorischen Volumenstrom zu bestimmen und eine Inspiration zu beginnen, wenn der dPEEP vorliegt.

Aus EP 3 769 668 A1 ist ein Beatmungsgerät bekannt. Das Beatmungsgerät ist eingerichtet, bei Erkennung einer inspiratorischen Flusslimitation und/oder einer exspiratorischen Flusslimitation und/oder eines intrinsischen PEEP und/oder einer ineffiziente Atemzugbemühung und/oder einer doppelten Atemzugbemühung eine Einstellung der Beatmung zu verändern. Die Einstellung der Beatmung ist ausgewählt aus IPAP und/oder EPAP und/oder Inspirationszeit und/oder Exspirationszeit und/oder Triggerempfindlichkeit.

Aus DE 696 23 400 T2 ist ein Beatmungssystem bekannt, das eingerichtet ist, ein optimales Verhältnis zwischen Inspirationszeit und Exspirationszeit zu finden.

Aus EP 0 127 905 A2 und EP 1 961 378 A1 sind weitere Beatmungsgeräte bekannt.

Aufgabe der vorliegenden Erfindung ist es, ein verbessertes Beatmungsgerät, insbesondere ein Beatmungsgerät mit einer besonders effizienten und Patienten-schonenden Beatmung, bereitzustellen.

Erfindungsgemäß wird zur Lösung dieser Aufgabe ein Beatmungsgerät zur maschinellen Beatmung eines Patienten vorgeschlagen, mit mindestens einer Sensoreinheit, einer Speichereinheit und einer Verarbeitungseinheit.

Die mindestens eine Sensoreinheit ist ausgebildet, einen Verlauf von Gasfluss-abhängigen Messwerten, insbesondere von Gasflüssen, im Beatmungskreis des Beatmungsgerätes zu messen und ein entsprechendes Sensorsignal auszugeben.

In der Speichereinheit ist eine Vielzahl von Beatmungsparametern eines aktuell vorliegenden Beatmungsmodus hinterlegt. Diese hinterlegten Beatmungsparameter indizieren zumindest eine Inspirationszeit der aktuell durch das Beatmungsgerät bereitgestellten Beatmung und eine darauffolgende Exspirationszeit für einen entsprechenden Atemzyklus. Ein Atemzyklus wird bekanntermaßen gebildet durch eine Kombination aus Inspirationszeit und darauffolgender Exspirationszeit.

Die Verarbeitungseinheit ist ausgebildet, das Sensorsignal zu empfangen und basierend auf dem Verlauf der Gasfluss-abhängigen Messwerte zumindest einen aktuellen endexspiratorischen Gasfluss zu bestimmen, wobei die Verarbeitungseinheit weiter ausgebildet ist, abhängig von einem Vergleich zwischen dem bestimmten aktuellen endexspiratorischen Gasfluss und einem unteren Schwellenwert und/oder einem oberen Schwellenwert ein Verhältnis zwischen Inspirationszeit und Exspirationszeit für den aktuell vorliegenden Beatmungsmodus zur Beatmung des Patienten zu verstellen, wobei die Dauer des endsprechenden Atemzyklus im Wesentlichen konstant bleibt.

Im Rahmen der Erfindung wurde erkannt, dass eine Phase ohne exspiratorischen Gasfluss oder mit wenig exspiratorischem Gasfluss dazu führt, dass während der Inspirationszeit unnötig schnell Atemgas zum Patienten geführt wird. So kann die Exspirationszeit um solch eine Phase geringen Gasflusses verkürzt werden, um die Inspirationszeit zu verlängern und dadurch einen Gradienten des inspiratorischen Gasflusses möglichst gering zu halten. Dies ist insbesondere vor dem Hintergrund vorteilhaft, dass eine Beatmung mit geringem Gradienten des inspiratorischen Gasflusses besonders Patienten-schonend ist.

Alternativ oder ergänzend kann erfindungsgemäß das Verhältnis zwischen Inspirationszeit und Exspirationszeit, insbesondere bei Patienten mit einer chronisch obstruktiven Erkrankung, durch ein Verlängern der Exspirationszeit verändert werden, falls der endexspiratorische Gasfluss oberhalb des oberen Schwellenwertes liegt. Hierdurch wird ein möglichst natürliches Ausatmen ermöglicht und der darauffolgende Druckgradient während der Inspirationszeit stellt für die entsprechende Patientengruppe meist kein Problem dar.

Weiterhin vorteilhaft an dem erfindungsgemäßen Beatmungsgerät ist die einfache Umsetzbarkeit der Erfindung. Die Veränderung des Verhältnisses zwischen Inspirationszeit und Exspirationszeit kann als reine Softwarelösung zur Anpassung bestehender Beatmungsgeräte realisiert werden. Dadurch ist die Umsetzung der Erfindung besonders kostengünstig möglich.

Angesichts einer großen Anzahl von am Markt etablierten Beatmungsmodi für Beatmungsgeräte kann die Erfindung vorteilhaft für eine Vielzahl verschiedener Beatmungsmodi angewendet werden, da meist eine Inspirationszeit und eine Exspirationszeit bei der Beatmung durch ein Beatmungsgerät vorliegen. Unter einem Beatmungsmodus ist ein Modus des Beatmungsgerätes zu verstehen, der auf eine Modus-spezifische Art und Weise die Beatmung des Patienten über die Steuerung oder Regelung entsprechender Beatmungsparameter kontrolliert.

Der Verlauf der Gasfluss-abhängigen Messwerte ist ein zeitabhängiger Verlauf, der kontinuierlich oder in diskreten Zeitschritten eine Entwicklung dieser Gasfluss-abhängigen Messwerte über die Zeit beschreibt.

Das Verhältnis zwischen Inspirationszeit und Exspirationszeit ist beispielsweise ein Quotient aus Inspirationszeit und Exspirationszeit, der verstellt wird durch eine Veränderung der Inspirationszeit und/oder durch eine Veränderung der Exspirationszeit. Vorzugsweise wird die Summe aus Inspirationszeit und Exspirationszeit, also die Dauer eines Atemzyklus, entsprechend einer vorgegebenen Atemfrequenz im Wesentlichen konstant gehalten.

Die Einheiten des erfindungsgemäßen Beatmungsgerätes sind vorzugsweise zumindest teilweise räumlich getrennt voneinander angeordnet. So ist die Sensoreinheit im Beatmungskreis des Beatmungsgerätes angeordnet. Der Beatmungskreis führt das Atemgas zum Patienten und wieder zurück zum Beatmungsgerät. Die Speichereinheit und die Verarbeitungseinheit werden vorzugsweise in einem zentralen Gehäuse des Beatmungsgerätes angeordnet und werden besonders bevorzugt von einem gemeinsamen Prozessor angesteuert. Die Einheiten sind dabei zumindest auf Softwareebene voneinander getrennt.

Der untere Schwellenwert und der obere Schwellenwert können vorbestimmte hinterlegte Werte für den endexspiratorischen Gasfluss oder einer damit korrelierten Systemgröße sein. Alternativ oder ergänzend kann mindestens einer der Schwellenwerte fortlaufend anhand aktuell bestimmter Werte, wie beispielsweise der Gasfluss-abhängigen Messwerte, neu bestimmt werden.

Nachfolgend werden bevorzugte Ausführungsformen des erfindungsgemäßen Beatmungsgerätes beschrieben.

In einer besonders bevorzugten Ausführungsform ist die Verarbeitungseinheit ausgebildet, die Exspirationszeit zu verkürzen, falls der aktuelle endexspiratorische Gasfluss unterhalb des unteren Schwellenwerts liegt. In diesem Fall ist der aktuelle endexspiratorische Gasfluss so gering, dass aus physiologischer Sicht schon früher eine nächste Inspirationsphase hätte starten können. Daher wird das Beatmungsgeräts erfindungsgemäß in dieser Ausführungsform die Exspirationszeit im Rahmen des nächsten Atemzyklus verkürzen. Besonders bevorzugt wird hierbei die vorbestimmte Atemfrequenz beibehalten, sodass die Verkürzung der Exspirationszeit zu einer entsprechenden Verlängerung der Inspirationszeit führt. Besonders bevorzugt wird ebenfalls das Tidalvolumen konstant gehalten, sodass während der nun längeren Inspirationszeit ein besonders geringer Druckgradient, also auch ein besonders geringer Gasflussgradient, und mithin eine besonders schonende Beatmung des Patienten möglich ist.

In einer besonders vorteilhaften Variante der vorhergehenden Ausführungsform stellt die Verarbeitungseinheit eine Zeit-Regelung bereit, bei der der aktuelle endexspiratorische Gasfluss als Messgröße der Zeit-Regelung und die Inspirationszeit als Stellgröße der Zeit-Regelung fungieren, und wobei der untere Schwellenwert einen Soll-Wert der Messgröße darstellt. Über eine derartige Regelung kann besonders zuverlässig sichergestellt werden, dass die Dauer der Inspirationszeit derart erfindungsgemäß verstellt wird, dass der endexspiratorische Gasfluss sich im Bereich des unteren Schwellenwertes einpegelt. Der endexspiratorische Gasfluss führt unmittelbar zu einem intrinsischen endexspiratorischen Gasdruck (intrinsic PEEP), auf den in dieser Variante eingepegelt wird. Da der Druck im Wesentlichen quadratisch vom Gasfluss abhängig ist, sollte dabei ein niedriger unterer Schwellenwert für den Gasfluss zu einem im Wesentlichen verschwindenden intrinsischen PEEP führen. Besonders bevorzugt wird zusammen mit dem Verändern der Inspirationszeit die Exspirationszeit ebenfalls verändert, sodass die Summe aus Inspirationszeit und Exspirationszeit, also die Dauer eines Atemzyklus, konstant bleibt. Das Vorsehen derartiger Regelungsglieder innerhalb eines Steuergerätes ist dem Fachmann bekannt und wird daher im Folgenden nicht detailliert erläutert. Insbesondere ist bekannt, wie eine solche Regelung ohne ein starkes Schwingungsverhalten der Parameter und mithin eine Beeinträchtigung der Beatmung des Patienten ausgeführt werden kann.

In einer zur vorhergehenden Variante alternativen oder ergänzenden Variante der beschriebenen Ausführungsformen ist die Verarbeitungseinheit ausgebildet, die Exspirationszeit zu verlängern, falls der aktuelle endexspiratorische Gasfluss oberhalb des oberen Schwellenwerts liegt. In dieser Variante der Ausführungsform ist ein Gasfluss-Bereich zwischen unterem Schwellenwert und oberem Schwellenwert vorgegeben, in dem der endexspiratorische Gasfluss liegen sollte. Falls der endexspiratorische Gasfluss außerhalb dieses Bereiches liegt, wird die Exspirationszeit so verlängert oder verkürzt, dass wieder ein endexspiratorischer Gasfluss innerhalb dieses Bereiches erreicht wird. Falls der endinspiratorische Gasfluss über dem oberen Schwellenwert liegt, sorgt eine Verlängerung der Exspirationszeit dafür, dass der endexspiratorische Gasfluss sinkt und mithin auch bei einer ausreichenden Verlängerung der Exspirationszeit unterhalb des oberen Schwellenwertes sinkt. Entsprechend liegt der obere Schwellenwert erfindungsgemäß grundsätzlich über dem unteren Schwellenwert. Eine derartige Verlängerung der Exspirationszeit kann beispielsweise bei Patienten mit chronisch obstruktiver Erkrankung vorteilhaft sein, die über einen vergleichsweise langen Zeitraum ausatmen müssen, ehe wieder das Einatmen beginnen kann. Bei diesen Patienten ist der dadurch vergrößerte Druckgradient während der Inspirationszeit meist nicht schädlich für das Patientenwohl.

Erfindungsgemäß ist die Verarbeitungseinheit des Beatmungsgerätes ausgebildet, die Exspirationszeit und die Inspirationszeit stets derart zu verstellen, dass die Dauer des endsprechenden Atemzyklus im Wesentlichen konstant bleibt. Hierdurch wird vorteilhaft sichergestellt, dass eine vorzugsweise vorgegebene Atemfrequenz der Beatmung durch das erfindungsgemäße Beatmungsgerät im Wesentlichen konstant gehalten wird. Da es sich bei der Atemfrequenz um einen besonders relevanten vorgegebenen Beatmungsparameter fast jedes Beatmungsmodus handelt, ist das Beibehalten einer Dauer des Atemzyklus im Rahmen dieser Ausführungsform besonders vorteilhaft.

In einer weiteren Ausführungsform des erfindungsgemäßen Beatmungsgerätes sind der untere Schwellenwert und/oder der obere Schwellenwert abhängig von dem gemessenen Verlauf der Gasfluss-abhängigen Messwerte. Durch die Abhängigkeit von dem gemessenen Verlauf sind die beiden Schwellenwerte in dieser Ausführungsform besonders vorteilhaft zumindest teilweise abhängig von der Beatmungscharakteristik des spezifischen Patienten. Besonders bevorzugt sind der untere Schwellenwert und/oder der obere Schwellenwert abhängig von einem maximalen Gasfluss, insbesondere einem maximalen exspiratorischen Gasfluss. So kann der untere Schwellenwert beispielsweise zwischen 0,1 % und 5 %, vorzugsweise zwischen 0,5 % und 3 %, insbesondere bei 2 % des maximalen exspiratorischen Gasflusses liegen. Der exspiratorische Gasfluss ist grundsätzlich derjenige Gasfluss eines Atemgases, welches der Patient während der Exspirationszeit ausgeatmet hat, wohingegen der inspiratorische Gasfluss derjenige Gasfluss eines Atemgases ist, welches der Patient während der Inspirationszeit einatmet. Somit wird der maximale exspiratorische Gasfluss aus mindestens einem der Gasfluss-abhängigen Messwerte aus dem gemessenen Verlauf bestimmt.

Die Verarbeitungseinheit ist ferner ausgebildet, abhängig von dem Verstellen des Verhältnisses zwischen Inspirationszeit und Exspirationszeit einen Anstieg eines Gasdrucks des Atemgases zu Beginn der Inspirationszeit und/oder einen Maximaldruck des Atemgases während der Inspirationszeit zu verstellen.

Durch das Verstellen des Anstiegs des Gasdrucks und/oder des Maximaldrucks wird die bereitgestellte Beatmungscharakteristik vorteilhaft an die verstellte Inspirationszeit angepasst. So kann beispielsweise eine längere Exspirationszeit dazu führen, dass der Maximaldruck reduziert wird, sodass die Beatmung während der Inspirationszeit schonender für den Patienten ist. Alternativ oder ergänzend kann eine längere Inspirationszeit dazu führen, dass der Anstieg des Gasdrucks, insbesondere der Anstieg des Gasdrucks zu Beginn der Inspirationszeit, reduziert wird. Dadurch wird der Gradient des Gasdruckes und somit auch der Gradient des Gasflusses reduziert, was bekanntermaßen die Beatmung durch das Beatmungsgerät angenehmer für den Patienten macht. Folglich werden in dieser Ausführungsform Beatmungsparameter besonders vorteilhaft an den aktuellen Zustand der Beatmung des Patienten angepasst, um eine besonders effektive und vorzugsweise eine besonders Patienten-schonende Beatmung zu ermöglichen.

In einer vorteilhaften Ausführungsform ist die Verarbeitungseinheit weiter ausgebildet, einen aktuellen endinspiratorischen Gasfluss über den Verlauf der Gasfluss-abhängigen Messwerte zu bestimmen und abhängig von dem aktuellen endinspiratorischen Gasfluss eine Rampendauer des Anstiegs des Gasdrucks des Atemgases zu Beginn der Inspirationszeit zu verstellen. Die Rampendauer ist hierbei dasjenige Zeitintervall zu Beginn der Inspirationszeit, zu dem der inspiratorische Gasfluss zu dem Patienten linear über die Zeit, also rampenförmig, bis zu einem maximalen inspiratorischen Gasfluss erhöht wird. Durch das Auswerten des aktuellen endinspiratorischen Gasflusses wird vorteilhaft ein weiterer Kennwert aus dem gemessenen Verlauf der Gasfluss-abhängigen Messwerte zur Verbesserung der Beatmung durch das erfindungsgemäße Beatmungsgerät verwendet. Über den endinspiratorischen Gasfluss kann festgestellt werden, ob zum Ende der Exspirationszeit noch ein Zeitbereich übrig ist, in dem keine Zuführung von Atemgas zu dem Patienten erfolgt, ehe die Exspirationszeit des entsprechenden Atemzyklus beginnt. In einer bevorzugten Variante dieser Ausführungsform ist die Verarbeitungseinheit ausgebildet, die Rampendauer zu verlängern, falls der aktuelle endinspiratorische Gasfluss unterhalb eines unteren inspiratorischen Schwellenwertes liegt, und gleichzeitig eine restliche Plateaudauer der Inspirationszeit zu verkürzen, so dass die Inspirationszeit, also die gesamte Inspirationszeit, unabhängig von dem endinspiratorischen Gasfluss ist. Dadurch kann sichergestellt werden, dass während der Inspirationszeit das dem Patienten zuzuführende Atemgas, vorzugsweise mit einem vorbestimmten Atemgasvolumen, möglichst Patienten-schonend bereitgestellt werden kann. Der untere inspiratorische Schwellenwert kann ein fest vorbestimmter Wert sein. Alternativ oder ergänzend kann der inspiratorische Schwellenwert abhängig sein von dem gemessenen Verlauf der Gasfluss-abhängigen Messwerte. Durch die Abhängigkeit von dem gemessenen Verlauf ist der Schwellenwert in dieser Ausführungsform besonders vorteilhaft zumindest teilweise abhängig von der Beatmungscharakteristik des spezifischen Patienten. Besonders bevorzugt ist der untere inspiratorische Schwellenwert abhängig von einem maximalen Gasfluss, insbesondere einem maximalen inspiratorischen Gasfluss. So kann der untere inspiratorische Schwellenwert beispielsweise zwischen 0,1 % und 5 %, vorzugsweise zwischen 0,5 % und 3 %, insbesondere bei 2 % des maximalen inspiratorischen Gasflusses liegen.

In einer besonders bevorzugten Variante der vorhergehenden Ausführungsform stellt die Verarbeitungseinheit eine Rampen-Regelung bereit, bei der der aktuelle endinspiratorische Gasfluss als Messgröße der Rampenregelung und die Rampendauer als Stellgröße der Rampenregelung fungieren, und wobei der untere inspiratorische Schwellenwert einen Soll-Wert der Messgröße darstellt. In dieser Variante kann über die Rampendauer vorteilhaft sichergestellt werden, dass sich der inspiratorische Gasfluss im Bereich des unteren inspiratorischen Schwellenwertes einpegelt. Ein Verringern der Rampendauer sorgt für eine Reduzierung des endinspiratorischen Gasflusses, wohingegen eine Verlängerung der Rampendauer den endinspiratorischen Gasfluss erhöht.

In einer zur vorhergehenden Variante alternativen oder ergänzenden Variante ist die Verarbeitungseinheit ausgebildet, die Rampendauer zu verkürzen, falls der aktuelle endinspiratorische Gasfluss oberhalb eines oberen inspiratorischen Schwellenwertes liegt, und gleichzeitig eine restliche Plateaudauer der Inspirationszeit zu verlängern, so dass die Inspirationszeit unabhängig von dem endinspiratorischen Gasfluss ist. In dieser Variante ist das erfindungsgemäße Beatmungsgerät ausgebildet, über den vorbestimmten oberen inspiratorischen Schwellenwert und den vorbestimmten unteren inspiratorischen Schwellenwert durch das Regeln der Rampendauer einen Bereich für den inspiratorischen Gasfluss festzulegen, in dem das Beatmungsgerät betrieben wird.

Der vorbestimmte obere inspiratorische Schwellenwert und/oder der vorbestimmte untere inspiratorische Schwellenwert sind vorzugsweise abhängig von dem gemessenen Verlauf der Gasfluss-abhängigen Messwerte. Besonders bevorzugt ist der untere inspiratorische Schwellenwert und/oder der obere inspiratorische Schwellenwert abhängig von einem aus den Gasfluss-abhängigen Messwerten bestimmten maximalen inspiratorischen Gasfluss während der Inspirationszeit der bereitgestellten Beatmung.

In einer besonders bevorzugten Ausführungsform ist die Verarbeitungseinheit weiter ausgebildet, die Exspirationszeit nur dann zu verlängern und/oder die Rampendauer bei konstantem Maximaldruck nur dann zu verlängern, wenn dadurch ein mittlerer Beatmungsdruck über den entsprechenden Atemzyklus nicht unterhalb eines vorgegebenen unteren Mitteldruckschwellenwertes liegt. In dieser Ausführungsform wird vorteilhaft sichergestellt, dass der vorgegebene unteren Mitteldruckschwellenwertes nicht unterschritten wird. Dies ist insbesondere deswegen vorteilhaft, weil der untere Mitteldruckschwellenwert ein besonders relevanter Parameter bei der Beatmung eines Patienten ist, sodass das Beatmungsgerät zuverlässig sicherstellen sollte, dass der durch das Verstellen der Exspirationszeit und/oder der Rampendauer real am Patienten vorliegende Mitteldruck stets oberhalb dieses Schwellenwertes liegt. So erlaubt das erfindungsgemäße Beatmungsgerät in dieser Ausführungsform neben der Patienten-schonenden Beatmung über eine Anpassung der Exspirationszeit und/oder die Rampendauer eine Einhaltung des vorgegebenen unteren Mitteldruckschwellenwertes und stellt somit einen besonders Patienten-schonenden Erfolg der Beatmung des Patienten sicher. Die Bestimmung des mittleren Beatmungsdruckes ist üblich bei für den Fachmann bekannten Beatmungsgeräten und verläuft typischerweise über eine Aufsummierung von Beatmungsdrücken über die Zeit.

In einer weiteren Ausführungsform weist das erfindungsgemäße Beatmungsgerät weiterhin eine Benutzerschnittstelle auf, die ausgebildet ist, eine Benutzereingabe zu empfangen. Die Benutzereingabe indiziert beispielsweise einen durch das Beatmungsgerät einstellbaren Bereich für das Verhältnis zwischen Inspirationszeit und Exspirationszeit. Das Verhältnis zwischen Inspirationszeit und Exspirationszeit liegt vorzugsweise zwischen einem Verhältnis von 1 zu 1 und einem Verhältnis von 1 zu 5, wie beispielsweise zwischen einem Verhältnis von 1 zu 1 in einem Verhältnis von 1 zu 3. Die Benutzereingabe kann alternativ oder ergänzend den zu verwendenden Beatmungsmodus indizieren. Alternativ oder ergänzend kann die Benutzereingabe einen zu hinterlegenden Beatmungsparameter indizieren, wie etwa eine Atemfrequenz, ein Digitalvolumen, einen Maximaldruck des Atemgases während der Inspirationszeit, einen Anstieg des Gasdrucks zu Beginn der Inspirationszeit, den unteren Schwellenwert für den endexspiratorischen Gasfluss, den unteren Mitteldruckschwellenwertes, oder dergleichen.

Es wird weiter ein Verfahren zum Betreiben eines Beatmungsgerätes zur maschinellen Beatmung eines Patienten beschrieben, aufweisend die Schritte:

- Messen eines Verlaufs von Gasfluss-abhängigen Messwerten im Beatmungskreis des Beatmungsgerätes und Ausgeben eines entsprechenden Sensorsignals;
- Hinterlegen einer Vielzahl von Beatmungsparametern eines aktuell vorliegenden Beatmungsmodus, wobei diese hinterlegten Beatmungsparameter zumindest eine Inspirationszeit der aktuell durch das Beatmungsgerät bereitgestellten Beatmung und eine darauffolgende Exspirationszeit für einen entsprechenden Atemzyklus indizieren;
- Empfangen des Sensorsignals und Bestimmen zumindest eines aktuellen endexspiratorischen Gasflusses basierend auf dem Verlauf der Gasfluss-abhängigen Messwerte; und
- Verstellen eines Verhältnisses zwischen Inspirationszeit und Exspirationszeit für den aktuell vorliegenden Beatmungsmodus zur Beatmung des Patienten abhängig von einem Vergleich zwischen dem bestimmten aktuellen endexspiratorischen Gasfluss und einem unteren Schwellenwert und/oder einem oberen Schwellenwert.

Das Verfahren wird durch das erfindungsgemäße Beatmungsgerät ausgeführt und umfasst daher sämtliche im Rahmen des Beatmungsgerätes genannten Vorteile. Weiterhin kann das Verfahren durch Merkmale entsprechend der beschriebenen Ausführungsformen des erfindungsgemäßen Beatmungsgerätes ergänzt werden.

Besonders vorteilhaft erlaubt das Verfahren ein dynamisches Verstellen des Verhältnisses zwischen Inspirationszeit und Exspirationszeit. Hierdurch kann berücksichtigt werden, dass der aktuelle endexspiratorische Gasfluss besonders hoch oder besonders niedrig ist, so dass die Inspirationszeit und/oder die Exspirationszeit entsprechend angepasst werden kann, um eine besonders Patienten-schonende Beatmung während der Inspirationszeit zu ermöglichen und gleichzeitig Phasen ohne inspiratorischen und exspiratorischen Gasfluss zu vermeiden.

Die Schritte des Verfahrens können in einer anderen als der dargestellten Reihenfolge ausgeführt werden. Insbesondere das Hinterlegen der Vielzahl von Beatmungsparametern kann auch vor dem Messen des Verlaufs von Gasfluss-abhängigen Messwerten erfolgen.

Vorzugsweise vergehen zwischen der Messung des Verlaufs der Gasfluss-abhängigen Messwerte und dem entsprechenden Verstellen des Verhältnisses zwischen Inspirationszeit und Exspirationszeit weniger als 20 Sekunden, insbesondere weniger als 10 Sekunden, besonders bevorzugt weniger als 2 Sekunden. So kann im Wesentlichen in Echtzeit eine Anpassung von Inspirationszeit und Exspirationszeit erfolgen, sodass die Beatmung des Patienten besonders schnell an den aktuellen Zustand der patientenspezifischen Atmung angepasst werden kann.

In einer Ausführungsform des beschriebenen Verfahrens wird über eine Anzahl von zurückliegenden endexspiratorischen Gasflüssen, etwa über die letzten beiden endexspiratorischen Gasflüsse gemittelt, um den mittleren Wert mit dem unteren Schwellenwert und/oder dem oberen Schwellenwert zu vergleichen. In dieser Ausführungsform wird der Vergleich dadurch abhängig von dem aktuellen endexspiratorischen Gasfluss ausgeführt, dass der bestimmte gemittelte Wert von dem aktuellen endexspiratorischen Gasfluss abhängig ist. In einer alternativen Ausführungsform wird das Verhältnis zwischen Inspirationszeit und Exspirationszeit ausschließlich basierend auf dem letzten bestimmten aktuellen endexspiratorischen Gasfluss verstellt.

Die Erfindung soll nun anhand von in den Figuren schematisch dargestellten, vorteilhaften Ausführungsbeispielen näher erläutert werden. Von diesen zeigen im Einzelnen:

Fig. 1    eine schematische Darstellung eines Ausführungsbeispiels eines erfindungsgemäßen Beatmungsgerätes;

Figs. 2, 3    ein jeweiliges Diagramm des Verlaufs von Gasfluss-abhängigen Messwerten, vor (Fig. 2) und nach (Fig. 3) einem Verstellen des Verhältnisses zwischen Inspirationszeit und Exspirationszeit in einem erfindungsgemäßen Ausführungsbeispiel;

Figs. 4, 5    ein jeweiliges Diagramm des Verlaufs von Gasfluss-abhängigen Messwerten, vor (Fig. 4) und nach (5) einem Verstellen einer Rampendauer eines Anstiegs eines Gasdrucks eines Atemgases in einem erfindungsgemäßen Ausführungsbeispiel;

Fig. 6    ein Flussdiagramm einer Regelung von Beatmungsparametern in einem erfindungsgemäßen Ausführungsbeispiel; und

Fig. 7    ein Flussdiagramm eines Ausführungsbeispiels eines Verfahrens.

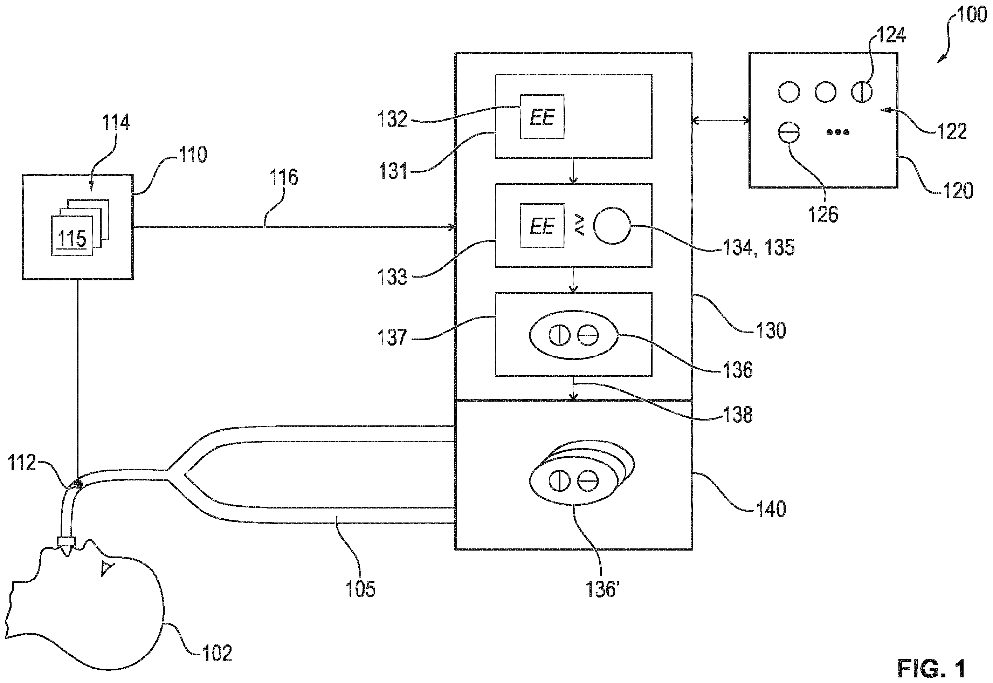

Fig. 1 zeigt eine schematische Darstellung eines Ausführungsbeispiels eines erfindungsgemäßen Beatmungsgerätes 100.

Das Beatmungsgerät 100 ist zur maschinellen Beatmung eines Patienten 102 ausgebildet. Dabei umfasst das Beatmungsgerät 100 mindestens eine Sensoreinheit 110, eine Speichereinheit 120 und eine Verarbeitungseinheit 130.

Die mindestens eine Sensoreinheit 110 ist ausgebildet, einen Verlauf 114 von Gasfluss-abhängigen Messwerten 115 im Beatmungskreis 105 des Beatmungsgerätes 100 zu messen. Hierfür weist die Sensoreinheit 110 in dem dargestellten Ausführungsbeispiel einen Sensorkopf 112 auf, der innerhalb des Beatmungskreises 105, insbesondere nahe einem am Patienten 102 befindlichen Tubus, angeordnet ist. Zudem ist die Sensoreinheit 110 dazu ausgebildet, ein entsprechendes Sensorsignal 116 auszugeben. Bei den Gasfluss-abhängigen Messwerten 115 handelt es sich vorliegend um einen gemessenen Gasfluss.

Alternativ oder ergänzend wird in einem nicht dargestellten Ausführungsbeispiel der Gasdruck als Gasfluss-abhängiger Messwert gemessen.

Die Speichereinheit 120 umfasst einen Speicher, in dem eine Vielzahl von Beatmungsparametern 122 eines aktuell vorliegenden Beatmungsmodus des Beatmungsgerätes 100 hinterlegt ist. Die Vielzahl von hinterlegten Beatmungsparametern 122 indiziert zumindest eine Inspirationszeit 124 der aktuell durch das Beatmungsgerät 100 bereitgestellten Beatmung und eine darauffolgende Exspirationszeit 126 für einen entsprechenden Atemzyklus. In dem dargestellten Ausführungsbeispiel ist in der Speichereinheit 120 zumindest die Inspirationszeit 124, die darauffolgende Exspirationszeit 126, ein bereitzustellendes Vitalvolumen, eine Atemfrequenz und ein Maximaldruck während der Inspiration hinterlegt. Die Speichereinheit 120 kann hierfür mit einer Benutzerschnittstelle zur Eingabe von Beatmungsparametern und/oder mit einer Steuereinheit 140 des Beatmungsgerätes 100 zum Vorgeben der aktuellen Beatmungsparameter und/oder mit einem Netzwerk, insbesondere einem Krankenhausnetzwerk, zum Bereitstellen der anzuwendenden Beatmungsparameter verbunden sein. Aus Gründen der Übersichtlichkeit ist eine derartige Verbindung entsprechend einer dieser Varianten nicht in Fig. 1 dargestellt.

Die Verarbeitungseinheit 130 ist ausgebildet, das Sensorsignal 116 zu empfangen und basierend auf dem Verlauf 114 der Gasfluss-abhängigen Messwerte 115 zumindest einen aktuellen endexspiratorischen (EE) Gasfluss 132 zu bestimmen. Das Bestimmen des aktuellen endexspiratorischen Gasflusses 132 erfolgt in dem dargestellten Ausführungsbeispiel innerhalb eines ersten Moduls 131. ein damit verbundenes zweites Modul 133 der Verarbeitungseinheit 130 ist weiter ausgebildet, abhängig von einem Vergleich zwischen dem bestimmten aktuellen endexspiratorischen Gasfluss 132 und einem unteren Schwellenwert 134 und/oder einem oberen Schwellenwert 135 ein Verhältnis 136 zwischen Inspirationszeit 124 und Exspirationszeit 126 für den aktuell vorliegenden Beatmungsmodus zur Beatmung des Patienten 102 zu verstellen. Das Bestimmen des dann neu bereitzustellenden Verhältnisses 136 erfolgt in einem dritten Modul 137 der Verarbeitungseinheit 130. Die drei Module 131, 133 und 137 sind dabei zumindest derart auf Softwareebene getrennt, dass unterschiedliche Verarbeitungsschritte eines auszuführenden Programms unterschiedliche Module repräsentieren.

Die Verarbeitungseinheit 130 ist dabei dazu ausgebildet, dass Verhältnis durch eine entsprechende interne Ausgabe 138 von dem dritten Modul 137 an die Steuereinheit 140 des Beatmungsgerätes 100 auszugeben. Die Steuereinheit 140 wendet das neu bestimmte Verhältniss 136 durch ein Überschreiben des vorher genutzten Verhältnisses 136' zwischen Inspirationszeit 124 und Exspirationszeit 126 bei der Beatmung des Patienten 102 an.

Die Verarbeitungseinheit 130 ist in dem dargestellten Ausführungsbeispiel dazu ausgebildet, die Exspirationszeit 126 zu verkürzen, falls der aktuelle endinspiratorische Gasfluss 132 unterhalb des unteren Schwellenwertes 134 liegt. Hierdurch wird vorteilhaft sichergestellt, dass die Inspirationszeit 124 möglichst lang ist, damit bei der Beatmung ein möglichst geringer Druckgradient bei konstantem Tidalvolumen und konstanter Atemfrequenz vorliegt. Dadurch kann besonders Patienten-schonend beatmet werden.

Ein zu großer endexspiratorischer Gasfluss korreliert mit einem großen intrinsischen endexspiratorischen Druck (intrinsic PEEP), der bekanntermaßen ebenfalls vermieden werden sollte. Hierfür ist die Verarbeitungseinheit vorzugsweise dazu ausgebildet, die Exspirationszeit 126 zu verlängern, falls der aktuelle endexspiratorische Gasfluss 132 oberhalb des oberen Schwellenwertes 136 liegt.

Die Änderung des Verhältnisses zwischen Inspirationszeit 124 und Exspirationszeit 126 erfolgt dabei stets derart, dass die Dauer des endsprechenden Atemzyklus, also die Summe aus Inspirationszeit und Exspirationszeit, stets konstant bleibt. Hierdurch wird sichergestellt, dass eine vorzugsweise vorbestimmte Atemfrequenz im Wesentlichen während der Verstellung des Verhältnisses unverändert bleibt.

Der untere Schwellenwert und/oder der obere Schwellenwert sind in dem dargestellten Ausführungsbeispiel abhängig von dem gemessenen Verlauf 114 der Gasfluss-abhängigen Messwerte 115. So werden diese beiden Schwellenwerte basierend auf dem maximalen exspiratorischen Gasfluss während der Exspirationszeit 126 bestimmt. Vorzugsweise beträgt der untere Schwellenwert zwischen 0,5% und 5% des maximalen inspiratorischen Gasflusses, insbesondere zwischen 1% und 3% des maximalen exspiratorischen Gasflusses, besonders bevorzugt etwa 2% des maximalen exspiratorischen Gasflusses.

Schließlich ist die Verarbeitungseinheit 130 weiter ausgebildet, die Exspirationszeit 126 nur dann zu verlängern, wenn dadurch ein mittlerer Beatmungsdruck über den entsprechenden Atemzyklus nicht innerhalb eines vorbestimmten unteren Mitteldruckschwellenwertes liegt.

Die Verarbeitungseinheit ist zudem ausgebildet, einen Verlauf des Gasflusses während der Inspirationszeit 124 zu verstellen, beispielsweise durch ein Verändern des Anstiegs des Gasflusses und/oder durch ein Verändern eines maximalen Gasfluss während der Inspirationszeit 124 zu verstellen.

Die verschiedenen Einheiten des erfindungsgemäßen Beatmungsgerätes 100 können zumindest teilweise in einem gemeinsamen Gehäuse angeordnet sein. Dabei sind die verschiedenen Einheiten zumindest auf Softwareebene voneinander getrennt. Eine Kommunikation zwischen diesen Einheiten erfolgt kabelbasiert oder kabellos. Verschiedene Möglichkeiten eine derartige kabelbasierte oder kabellose Kommunikation zu realisieren sind dem Fachmann bekannt, sodass hierauf im Folgenden nicht detailliert eingegangen wird.

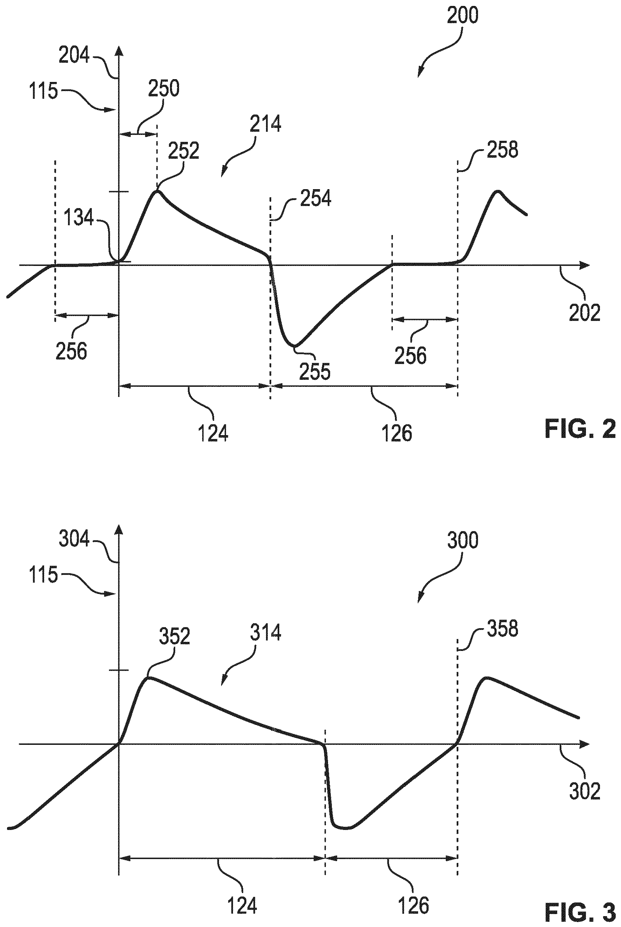

Die Figuren 2 und 3 zeigen ein jeweiliges Diagramm 200, 300 des Verlaufs 214, 314 von Gasfluss-abhängigen Messwerten 115, vor (Fig. 2) und nach (Fig. 3) einem Verstellen des Verhältnisses zwischen Inspirationszeit 124 und Exspirationszeit 126 in einem erfindungsgemäßen Ausführungsbeispiel.

Die Diagramme 200, 300 zeigen über der jeweiligen X-Achse 202, 302 die Zeit, die über etwas mehr als einen jeweiligen Atemzyklus dargestellt wird. Die Dauer eines Atemzyklus ist dabei zwischen 3 Sekunden und 12 Sekunden, insbesondere zwischen 5 Sekunden und 10 Sekunden. Am Koordinatenursprung des jeweiligen Diagramms 200, 300 liegt der Beginn des jeweils dargestellten Atemzyklus.

Über die jeweilige Y-Achse 204, 304 wird der Gasfluss-abhängige Messwert, also vorliegend der Gasfluss, dargestellt. Eine dargestellte Markierung liegt bei einem Wert von 25 L/min.

Der Gasfluss ist positiv während der Inspirationszeit 124 und negativ während der Exspirationszeit 126. Während der Inspirationszeit 124 steigt der Gasfluss während einer Rampendauer 250 im Wesentlichen linear an, ehe er nach einem maximalen inspiratorischen Gasfluss 252 wieder absinkt. Das Inspirationsende 254 der Inspirationszeit 124 weist idealerweise einen positiven Gasfluss nahe 0 L/min auf. Nach dem Inspirationsende 254 beginnt die Exspirationszeit 126, bei der der Gasfluss bis zu einem maximalen exspiratorischen Gasfluss 255 in negativer Richtung der Y-Achse steigt, ehe der Gasfluss wieder auf einen Wert nahe 0 L/min sinkt. Vor der erfindungsgemäßen Anpassung der Exspirationszeit 126 gibt es in dem in Fig. 2 dargestellten beispielhaften Verlauf 214 einen ausgedehnten Bereich 256, an dem im Wesentlichen kein Gasfluss vorliegt, bis nach dem Exspirationsende 258 wieder die nächste Inspirationszeit 124 beginnt. Würde die Beatmung so weiterlaufen, gäbe es in jedem Atemzyklus den ausgedehnten Exspirationsbereich 256, der nicht effektiv zur Beatmung des entsprechenden Patienten beiträgt.

Erfindungsgemäß wird daher bei Vorliegen des Verlaufs 214 festgestellt, dass zum Exspirationsende 258 ein unterer Schwellenwert 134 unterschritten wird, sodass das Verhältnis zwischen Inspirationszeit 124 und Exspirationszeit 126 verstellt wird. Der untere Schwellenwert 134 beträgt in dem dargestellten Ausführungsbeispiel etwa 2 % des maximalen exspiratorischen Gasflusses 255.

Durch das Unterschreiten dieses unteren Schwellenwertes 134 wird die Exspirationszeit 126 verkürzt und gleichzeitig die Inspirationszeit 124 verlängert, wie in Fig. 3 dargestellt. Der gesamte Atemzyklus behält dabei seine Dauer, sodass das Exspirationsende 358 im Wesentlichen zum gleichen Zeitpunkt ist wie das Exspirationsende 258 aus Fig. 2. Der Verlauf der Exspiration ist in den Figuren 2 und 3 identisch, bis auf den Umstand, dass durch die Verkürzung der Exspirationszeit 126 der ausgedehnte Bereich 256 ohne oder mit wenig Gasfluss entfällt. Der Verlauf der Exspiration ist im Wesentlichen abhängig von dem bereitgestellten Tidalvolumen und der vorliegenden Patientenphysiologie und dadurch unabhängig von einer Veränderung des Verlaufs 214 des Gasflusses während der Inspirationszeit 124.

Das zuzuführende Gasvolumen wird angesichts der verlängerten Inspirationszeit 124 über einen längeren Zeitraum zugeführt, so dass der Gradient des Gasflusses, also die Änderung pro Zeit, geringer und dadurch angenehmer für den Patienten ist. Zudem ist in dem dargestellten Ausführungsbeispiel der maximale inspiratorische Gasfluss 352 geringer als der maximale inspiratorische Gasfluss 252 aus Fig. 2. Erfindungsgemäß wird abhängig von dem Verstellen des Verhältnisses zwischen Inspirationszeit und Exspirationszeit ein Anstieg des Gasdrucks des Atemgases zu Beginn der Inspirationszeit und/oder ein Maximaldruck des Atemgases während der Inspirationszeit verstellt.

Das Verstellen des Verhältnisses zwischen Inspirationszeit 124 und Exspirationszeit 126 kann über eine vorbestimmte Zeit-Regelung bereitgestellt sein, bei der der aktuelle endexspiratorische Gasfluss als Messgröße der Zeit-Regelung und die Inspirationszeit als Stellgröße der Zeit-Regelung fungieren, und wobei der untere Schwellenwert einen Soll-Wert der Messgröße darstellt. Dadurch wird beispielsweise sichergestellt, dass eine Verringerung der Exspirationszeit 126 nicht zu einem intrinsischen endexspiratorischen Gasdruck (instrinsic PEEP) führt.

Die weiteren bei der Beatmung zu berücksichtigenden Beatmungsparameter sind abhängig vom vorliegenden Beatmungsmodus dem Fachmann bekannt. Vorzugsweise ist zumindest die Atemrate der Beatmung vorgegeben.

Vorzugsweise ist zudem mindestens einer der folgenden Drücke vorgegeben: positiver endexspiratorischer Druck (PEEP), maximaler inspiratorischer Druck, mittlerer Atemdruck. Falls nur einer dieser Drücke vorgegeben ist, ist vorzugsweise ebenfalls das Tidalvolumen der Beatmung ein vorbestimmter Beatmungsparameter des vorliegenden Beatmungsmodus.

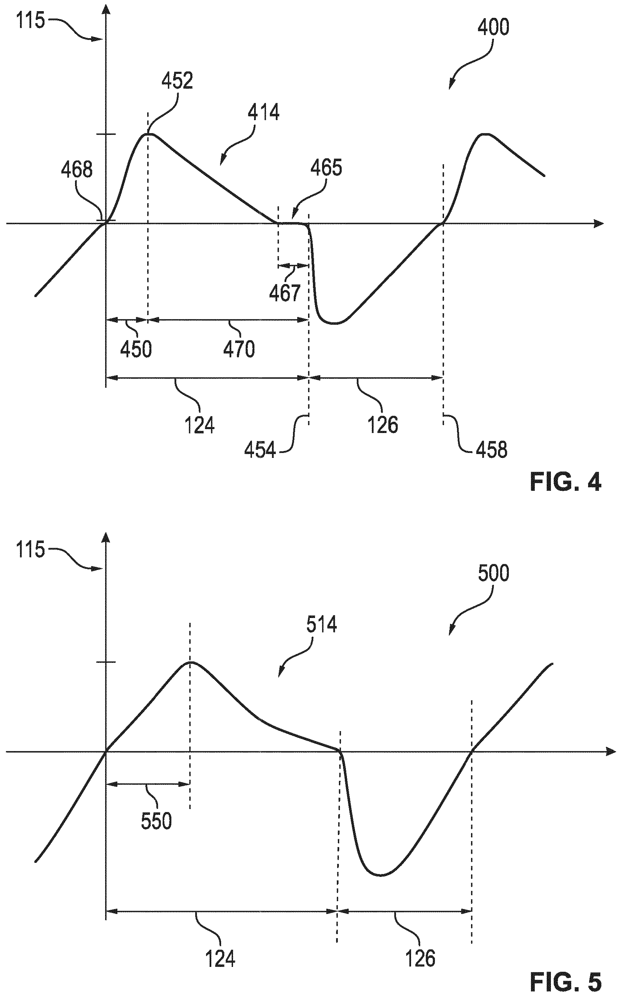

Figuren 4 und 5 zeigen ein jeweiliges Diagramm 400, 500 des Verlaufs 414, 514 von Gasfluss-abhängigen Messwerten 115, vor (Fig. 4) und nach (Fig. 5) einem Verstellen einer Rampendauer 450, 550 eines Anstiegs eines Gasdrucks eines Atemgases in einem erfindungsgemäßen Ausführungsbeispiel.

Die Achsen der Diagramme 400, 500 sind identisch zu den Achsen aus dem Diagramm 200, 300 ausgebildet.

In Fig. 4 unterscheidet sich der Verlauf 414 der Gasfluss-abhängigen Messwerte 115 von den Verläufen aus Fig. 2 und 3 dadurch, dass ein endinspiratorischer Gasfluss 465 über einen ausgedehnten Inspirationsbereich 467 im Wesentlichen 0 l/min beträgt. Zusätzlich zu dem Inspirationsende 454 und dem Exspirationsende 458 ist der Zeitpunkt des maximalen inspiratorischen Gasflusses 452 gekennzeichnet, der das Ende der Rampendauer 450 darstellt.

Das erfindungsgemäße Beatmungsgerät stellt fest, dass der endinspiratorische Gasfluss 465 über einem unteren inspiratorischen Schwellenwert 468 liegt und daher eine Verlängerung der Rampendauer 450 bei gleichbleibender Länge der Inspirationszeit 124 erfolgen muss. Hierbei wird die Plateaudauer 470, die die restliche Zeit der Inspirationszeit 124 darstellt, verkürzt. Die Bezeichnung Plateaudauer ergibt sich für dieses Zeitintervall, weil ein Druck innerhalb des Y-Stückes in diesem Zeitbereich konstant bleibt, während der restliche positive Gasfluss direkt zur Lunge des Patienten gelangt. Dadurch liegt in diesem Zeitbereich bekanntermaßen ein Druckplateau für einen typischerweise im Bereich des Y-Stückes angeordneten Drucksensor vor.

Der sich dabei ergebende Verlauf 514 ist in Fig. 5 dargestellt. Die Rampendauer 550 ist in etwa doppelt so lang wie die Rampendauer 450 aus Fig. 4, wohingegen der Verlauf 514 während der Exspirationszeit 126 im Wesentlichen unverändert geblieben ist. Die längere Rampendauer 550 wird für eine Verringerung des Anstiegs des Gasflusses und mithin eine schonende Beatmung des entsprechenden Patienten genutzt.

Das Beatmungsgerät aus diesem Ausführungsbeispiel würde bei einem endinspiratorischen Gasfluss oberhalb eines oberen inspiratorischen Schwellenwertes die Rampendauer auch entsprechend verkürzen.

Die geeignete Verstellung der Rampendauer und/oder der Inspirationszeit und/oder der Exspirationszeit wird durch ein iteratives Verstellen um vorbestimmte Zeitinkremente realisiert. Nach jedem Verstellvorgang wird der Einfluss auf den entsprechenden Verlauf der Messwerte berücksichtigt, um das weitere Verstellen zu steuern. Ein derartiges Vorgehen ist im Bereich der Regelungstechnik allgemein bekannt, sodass auf die Möglichkeiten der regelungstechnischen Realisierung im Folgenden nicht detailliert eingegangen wird.

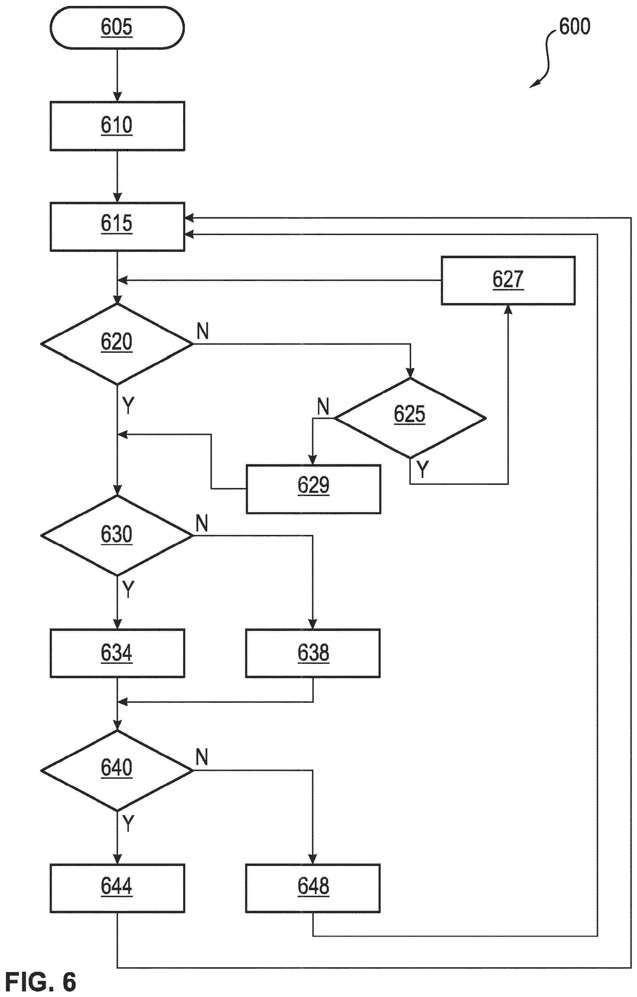

Fig. 6 zeigt ein Flussdiagramm einer Regelung 600 von Beatmungsparametern in einem erfindungsgemäßen Ausführungsbeispiel.

Die Regelung 600 veranschaulicht ein Ausführungsbeispiel für die erfindungsgemäßen Arbeitsschritte des Beatmungsgerätes beim Verstellen der Inspirationszeit und der Rampendauer.

Nach dem Beginn 605 der erfindungsgemäßen Regelung erfolgt die Eingabe und Hinterlegung der Vielzahl von Beatmungsparametern im Schritt 610. Danach wird die Beatmung des Patienten im Schritt 615 gestartet.

Während der Beatmung wird in diesem Ausführungsbeispiel vorteilhaft stets im Schritt 620 geprüft, ob der mittlere Beatmungsdruck über den entsprechenden Atemzyklus oberhalb eines vorgegebenen unteren Mitteldruckschwellenwertes liegt. Der mittlere Beatmungsdruck wird dabei beispielsweise durch ein Aufsummieren der gemessenen Gasfluss-abhängigen Messwerte, also beispielsweise der gemessenen Gasdrücke, mit einer entsprechenden Skalierung berechnet. Alternativ oder ergänzend kann der mittlere Beatmungsdruck MAP näherungsweise berechnet werden durch die folgende Berechnungsvorschrift:

$$MAP = PEEP + (T_I - \tfrac{1}{2} * T_{Ramp}) * RR * P$$

Hierbei ist $T_I$ die Inspirationszeit, die sich beispielsweise aus dem Verhältnis zwischen Inspirationszeit und Exspirationszeit und der vorgegebenen Atemfrequenz RR ergibt. Zudem ist $T_{Ramp}$ die Rampendauer und P der inspiratorische Drucksprung, der sich aus einem Quotienten aus Tidalvolumen und Lungenkapazität ergibt.

Wird im Schritt 620 festgestellt, dass der mittlere Beatmungsdruck über dem unteren Mitteldruckschwellenwert liegt, wird die Regelung 600 weiter mit dem Schritt 630 ausgeführt. Sollte der mittlere Beatmungsdruck unter dem unteren Mitteldruckschwellenwertes liegen, wird im Schritt 625 geprüft, ob die aktuelle Rampendauer größer als eine vorbestimmte minimale Rampendauer ist. Falls die Rampendauer größer als die vorbestimmte minimale Rampendauer ist, wird in dem Schritt 627 die Rampendauer inkrementell reduziert und der Schritt 620 wiederholt. Falls die Rampendauer kleiner oder gleich der minimalen Rampendauer ist, wird die Inspirationszeit im Schritt 629 verlängert, um den mittleren Beatmungsdruck zu erhöhen und es wird mit dem Schritt 630 fortgefahren.

Im Schritt 630 wird geprüft, ob der endexspiratorische Gasfluss größer als 2 % des maximalen exspiratorischen Gasfluss ist. Falls der endexspiratorische Gasfluss größer als 2 % des maximalen exspiratorischen Gasfluss ist, wird die Exspirationszeit im Schritt 634 verlängert, während die Summe aus Inspirationszeit und Exspirationszeit konstant gehalten wird, bis der endexspiratorische Gasfluss im Wesentlichen 2 % des maximalen exspiratorischen Gasflusses beträgt und/oder der mittlere Beatmungsdruck gleich dem unteren Mitteldruckschwellenwert ist. Falls der endexspiratorische Gasfluss größer als 2 % des maximalen exspiratorischen Gasflusses ist, wird im Schritt 638 die Exspirationszeit verkürzt, bis der endexspiratorische Gasfluss im Wesentlichen 2 % des maximalen exspiratorischen Gasflusses beträgt.

Danach wird der Schritt 640 ausgeführt, bei dem geprüft wird, ob der endinspiratorische Gasfluss größer als 2 % des maximalen inspiratorischen Gasflusses ist. Falls der endinspiratorische Gasfluss größer als 2 % des maximalen inspiratorischen Gasflusses ist, wird die Rampendauer im Schritt 644 reduziert, bis der endinspiratorische Gasfluss im wesentliche 2 % des maximalen inspiratorischen Gasflusses beträgt oder die Rampendauer im Wesentlichen der minimalen Rampendauer entspricht. Falls der endinspiratorische Gasfluss kleiner als 2 % des maximalen inspiratorischen Gasflusses ist, wird die Rampendauer im Schritt 648 erhöht, bis der endinspiratorische Gasfluss im Wesentlichen 2 % des maximalen inspiratorischen Gasflusses beträgt oder der mittlere Beatmungsdruck im Wesentlichen dem unteren Mitteldruckschwellenwertes entspricht.

Nach dem Ausführen des Schrittes 644 oder des Schrittes 648 werden die Schritte angefangen vom Durchführen der Beatmung 615 erneut ausgeführt.

In diesem Ausführungsbeispiel werden alle Aspekte der Regelung des erfindungsgemäßen Beatmungsgerätes erläutert. In alternativen Ausführungsbeispielen erfolgt nur die Regelung der Inspirationszeit oder nur eine Kombination der Regelung der Inspirationszeit und der Rampe oder nur eine Kombination der Regelung der Inspirationszeit und des mittleren Beatmungsdruckes.

Die Struktur der Regelung 600 visualisiert im Wesentlichen den iterativen Charakter der erfindungsgemäßen Regelung. Dabei erfolgt die inkrementelle Veränderung eines Wertes bei jedem Durchlaufen eines entsprechenden Schrittes dieser Regelung 600. Geeignete Startbedingungen für die Beatmung sind aus der medizinischen Praxis bekannt und werden daher im Folgenden nicht dargelegt.

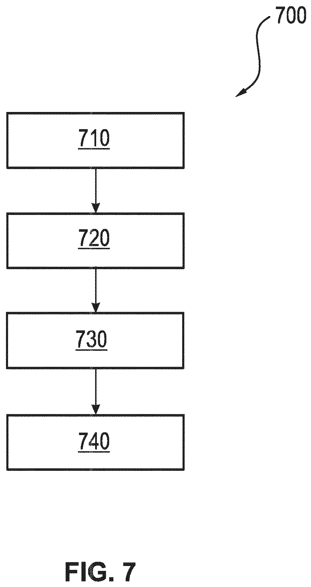

Fig. 7 zeigt ein Flussdiagramm eines Beispiels eines Verfahrens 700. Verglichen mit der in Fig. 6 dargestellten Regelung stellt das Verfahren 700 einen übergeordneten erfindungsgemäßen Ablauf von Verfahrensschritten dar. Es ist für den Fachmann aus der nachfolgenden Erläuterung klar verständlich, dass einzelne Verfahrensschritte des Verfahrens 700 mehrere Stufen der im Rahmen von Fig. 6 erläuterten Regelung 600 umfassen können.

Das Verfahren 700 ist zum Betreiben eines Beatmungsgerätes zur maschinellen Beatmung eines Patienten ausgebildet. Hierfür weist das Verfahren 700 die im Folgenden dargestellten Schritte auf. Ein erster Schritt 710 umfasst ein Messen eines Verlaufs von Gasfluss-abhängigen Messwerten im Beatmungskreis des Beatmungsgerätes und ein Ausgeben eines entsprechenden Sensorsignals.

Ein darauffolgender Schritt 720 umfasst ein Hinterlegen einer Vielzahl von Beatmungsparametern eines aktuell vorliegenden Beatmungsmodus, wobei diese hinterlegten Beatmungsparameter zumindest eine Inspirationszeit der aktuell durch das Beatmungsgerät bereitgestellten Beatmung und eine darauffolgende Exspirationszeit für einen entsprechenden Atemzyklus indizieren.

Ein weiterer Schritt 730 umfasst ein Empfangen des Sensorsignals und ein Bestimmen zumindest eines aktuellen endexspiratorischen Gasflusses basierend auf dem Verlauf der Gasfluss-abhängigen Messwerte.

Ein abschließender Schritt 740 umfasst ein Verstellen eines Verhältnisses zwischen Inspirationszeit und Exspirationszeit für den aktuell vorliegenden Beatmungsmodus zur Beatmung des Patienten abhängig von einem Vergleich zwischen dem bestimmten aktuellen endexspiratorischen Gasfluss und einem unteren Schwellenwert und/oder einem oberen Schwellenwert.

Die Reihenfolge der Verfahrensschritte 710 und 720 kann wechseln. So kann das Hinterlegen der Vielzahl von Beatmungsparametern bereits mit der Bereitstellung des Beatmungsgerätes erfolgen, aber auch während der Ausführung des Verfahrens durch ein Verändern von Beatmungsparametern erneut erfolgen.

Die weiteren Schritte 730 und 740 werden nach den beiden anfänglichen Schritten 710 und 720 ausgeführt. Dabei erfolgt das Bestimmen des aktuellen endexspiratorischen Gasflusses entsprechend Schritt 730 stets vor dem Verstellen des Verhältnisses zwischen Inspirationszeit und Exspirationszeit entsprechend Schritt 730.

Vorzugsweise erfolgt eine Ausführung der Schritte 730 und 740 zumindest nahezu in Echtzeit, sodass die Messwerte für einen Atemzyklus bereits bei der Veränderung des Verhältnisses zwischen Inspirationszeit und Exspirationszeit des darauffolgenden Atemzyklus berücksichtigt werden können. In einem alternativen oder ergänzenden Ausführungsbeispiel erfolgt das Verstellen des Verhältnisses zwischen Inspirationszeit und Exspirationszeit basierend auf einer Anzahl von Messwerten von zurückliegenden Atemzyklen, insbesondere basierend auf den letzten beiden Atemzyklen. Eine Berücksichtigung früherer Atemzyklen kann eine Zuverlässigkeit des erfindungsgemäßen Beatmungsgerätes verbessern, da beispielsweise aus einem Vergleich zwischen dem aktuellen Atemzyklus und dem zurückliegenden Atemzyklus bei besonders großer Abweichung auf Messfehler geschlossen werden kann. So kann beispielsweise eine Abweichung oberhalb eines Abweichungs-Schwellenwertes dazu führen, dass die aktuellen Messwerte nicht für das Verstellen des Verhältnisses zwischen Inspirationszeit und Exspirationszeit genutzt werden.

Die Schritte des beschriebenen Verfahrens 700 können mehrfach innerhalb des Verfahrens ausgeführt werden. So können beispielsweise mehrere aktuelle endexspiratorische Gasflüsse entsprechend dem Schritt 730 bestimmt werden, ehe ein Verstellen des Verhältnisses zwischen Inspirationszeit und Exspirationszeit entsprechend Schritt 740 erfolgt.

Vorzugsweise werden zumindest Teile des Verfahrens, wie etwa das Messen gemäß Schritt 710, das Bestimmen des aktuellen endexspiratorischen Gasflusses gemäß Schritt 730 und das Verstellen gemäß 740, für jeden Atemzyklus erneut ausgeführt. Dabei wird das Verstellen gemäß dem Schritt 740 erfindungsgemäß nur ausgeführt, wenn der Vergleich zwischen dem bestimmten aktuellen endexspiratorischen Gasfluss und dem entsprechenden Schwellenwert gemäß einer hinterlegten Vorschrift dies anzeigt.

Die verschiedenen Verfahrensschritte des Verfahrens 700 können an einem gemeinsamen Ort, beispielsweise durch ein gemeinsames Gerät ausgeführt werden. Dabei ist die Ausführung der einzelnen Schritte, beispielsweise durch einen gemeinsamen Prozessor, zumindest auf Softwareebene voneinander getrennt. Alternativ kann das Verfahren 700 zumindest teilweise an verschiedenen Orten ausgeführt werden.

## Bezugszeichenliste

100 Beatmungsgerät

102 Patient

105 Beatmungskreis

110 Sensoreinheit

112 Sensorkopf

114, 214, 314, 414, 514 Verlauf von Messwerten

115 Gasfluss-abhängiger Messwert

116 Sensorsignal

120 Speichereinheit

122 Beatmungsparameter

124 Inspirationszeit

126 Exspirationszeit

130 Verarbeitungseinheit

131 erstes Modul

132 endexspiratorischer Gasfluss

133 zweites Modul

134 unterer Schwellenwert

135 oberer Schwellenwert

136 Verhältnis zwischen Inspirations- und Exspirationszeit

136' zurückliegendes Verhältnis

137 drittes Modul

138 interne Ausgabe

140 Steuereinheit

200, 300, 400, 500 Diagramm

202, 302 X-Achse

204, 304 Y-Achse

250, 450, 550 Rampendauer

252, 352, 452 maximaler inspiratorischer Gasfluss

254, 454 Inspirationsende

255 maximaler exspiratorischer Gasfluss

256 ausgedehnter exspiratorischer Bereich

258, 358, 458 Exspirationsende

465 endinspiratorischer Gasfluss

467 ausgedehnter inspiratorischer Bereich

468 unterer inspiratorischer Schwellenwert

470 Plateaudauer

600 Regelung

605, 610, 615, 620, 625, 627, 629, 630, 634, 638, 640, 644, 648 Regelungsschritte

700 Verfahren

710, 720, 730, 740 Verfahrensschritte

**Patentansprüche**

1.  Beatmungsgerät (100) zur maschinellen Beatmung eines Patienten (102), mit

    - mindestens einer Sensoreinheit (110), die ausgebildet ist, einen Verlauf (114) von Gasfluss-abhängigen Messwerten (115) im Beatmungskreis (105) des Beatmungsgerätes (100) zu messen und ein entsprechendes Sensorsignal (116) auszugeben,
    - einer Speichereinheit (120),

        in der eine Vielzahl von Beatmungsparametern (122) eines aktuell vorliegenden Beatmungsmodus hinterlegt ist, wobei diese hinterlegten Beatmungsparameter (122) zumindest eine Inspirationszeit (124) der aktuell durch das Beatmungsgerät (100) bereitgestellten Beatmung und eine darauffolgende Exspirationszeit (126) für einen entsprechenden Atemzyklus indizieren,

    - einer Verarbeitungseinheit (130),

        die ausgebildet ist, das Sensorsignal (116) zu empfangen und basierend auf dem Verlauf (114) der Gasfluss-abhängigen Messwerte (115) zumindest einen aktuellen endexspiratorischen Gasfluss (132) zu bestimmen, wobei die Verarbeitungseinheit (130) weiter ausgebildet ist, abhängig von einem Vergleich zwischen dem bestimmten aktuellen endexspiratorischen Gasfluss (132) und einem unteren Schwellenwert (134) und/oder einem oberen Schwellenwert (135) ein Verhältnis (136) zwischen Inspirationszeit (124) und Exspirationszeit (126) für den aktuell vorliegenden Beatmungsmodus zur Beatmung des Patienten (102) zu verstellen, wobei die Dauer des endsprechenden Atemzyklus im Wesentlichen konstant bleibt, **dadurch gekennzeichnet, dass** die Verarbeitungseinheit (130) ausgebildet ist, abhängig von dem Verstellen des Verhältnisses (136) zwischen Inspirationszeit (124) und Exspirationszeit (126) einen Anstieg eines Gasdrucks des Atemgases zu Beginn der Inspirationszeit (124) und/oder einen Maximaldruck des Atemgases während der Inspirationszeit (124) zu verstellen.

2. Beatmungsgerät (100) gemäß Anspruch 1, wobei die Verarbeitungseinheit (130) ausgebildet ist, die Exspirationszeit (126) zu verkürzen, falls der aktuelle endexspiratorische Gasfluss (132) unterhalb des unteren Schwellenwerts (134) liegt.

3. Beatmungsgerät (100) gemäß Anspruch 1 und 2,

   wobei die Verarbeitungseinheit (130) eine Zeit-Regelung bereitstellt, bei der der aktuelle endexspiratorische Gasfluss (132) als Messgröße der Zeit-Regelung und die Inspirationszeit (124) als Stellgröße der Zeit-Regelung fungieren, und
   wobei der untere Schwellenwert (134) einen Soll-Wert der Messgröße darstellt.

4. Beatmungsgerät (100) gemäß Anspruch 1 und 2, wobei die Verarbeitungseinheit (130) ausgebildet ist, die Exspirationszeit (126) zu verlängern, falls der aktuelle endexspiratorische Gasfluss (132) oberhalb des oberen Schwellenwerts (135) liegt.

5. Beatmungsgerät (100) gemäß mindestens einem der vorhergehenden Ansprüche, wobei der untere Schwellenwert (134) und/oder der obere Schwellenwert (135) abhängig sind von dem gemessenen Verlauf (114) der Gasfluss-abhängigen Messwerte (115).

6. Beatmungsgerät (100) gemäß Anspruch 5, wobei der untere Schwellenwert (134) und/oder der obere Schwellenwert (135) abhängig sind von einem aus den Gasfluss-abhängigen Messwerten (115) bestimmten maximalen exspiratorischen Gasfluss (255) während der Exspirationszeit (126) der bereitgestellten Beatmung.

7. Beatmungsgerät (100) gemäß mindestens einem der vorherigen Ansprüche, wobei die Verarbeitungseinheit (130) weiter ausgebildet ist, den Anstieg des Gasdrucks zu Beginn der Inspirationszeit (124) zu reduzieren und/oder den Maximaldruck des Atemgases zu reduzieren, falls die Inspirationszeit (124) verlängert wird.

8. Beatmungsgerät (100) gemäß mindestens einem der vorhergehenden Ansprüche, wobei die Verarbeitungseinheit (130) weiter ausgebildet ist, einen aktuellen endinspiratorischen Gasfluss (465) über den Verlauf (114) der Gasfluss-abhängigen Messwerte (115) zu bestimmen und abhängig von dem aktuellen endinspiratorischen Gasfluss (465) eine Rampendauer (450) des Anstiegs des Gasdrucks des Atemgases zu Beginn der Inspirationszeit (124) zu verstellen.

9. Beatmungsgerät (100) gemäß Anspruch 8, wobei die Verarbeitungseinheit (130) ausgebildet ist, die Rampendauer (450) zu verlängern, falls der aktuelle endinspiratorische Gasfluss (465) unterhalb eines unteren inspiratorischen Schwellenwertes (468) liegt, und gleichzeitig eine restliche Plateaudauer (470) der Inspirationszeit (124) zu verkürzen, so dass die Inspirationszeit (124) unabhängig von dem endinspiratorischen Gasfluss (465) ist.

10. Beatmungsgerät (100) gemäß Anspruch 8 und 9,

    wobei die Verarbeitungseinheit (130) eine Rampen-Regelung bereitstellt, bei der der aktuelle endinspiratorische Gasfluss (465) als Messgröße der Rampenregelung und die Rampendauer (450) als Stellgröße der Rampenregelung fungieren, und
    wobei der untere inspiratorische Schwellenwert (468) einen Soll-Wert der Messgröße darstellt.

11. Beatmungsgerät (100) gemäß Anspruch 8 und 9, wobei die Verarbeitungseinheit (130) ausgebildet ist, die Rampendauer (450) zu verkürzen, falls der aktuelle endinspiratorische Gasfluss (465) oberhalb eines oberen inspiratorischen Schwellenwertes liegt, und gleichzeitig eine restliche Plateaudauer (470) der Inspirationszeit (124) zu verlängern, so dass die Inspirationszeit (124) unabhängig von dem endinspiratorischen Gasfluss (465) ist.

12. Beatmungsgerät (100) gemäß mindestens einem der vorhergehenden Ansprüche, wobei die Verarbeitungseinheit (130) weiter ausgebildet ist, die Exspirationszeit (126) nur dann zu verlängern und/oder die Rampendauer (450) bei konstantem Maximaldruck nur dann zu verlängern, wenn dadurch ein mittlerer Beatmungsdruck über den entsprechenden Atemzyklus nicht unterhalb eines vorgegebenen unteren Mitteldruckschwellenwertes liegt.

**Claims**

1. Ventilator (100) for mechanical ventilation of a patient (102), comprising

   - at least one sensor unit (110), which is designed to measure a profile (114) of gas flow-dependent measured values (115) in the ventilation circuit (105) of the ventilator (100) and to output a corresponding sensor signal (116),
   - a storage unit (120),

in which a plurality of ventilation parameters (122) of a currently present ventilation mode are stored,

these stored ventilation parameters (122) indicating at least one inspiratory time (124) of the ventilation currently provided by the ventilator (100) and a subsequent expiratory time (126) for a corresponding respiratory cycle,

- a processing unit (130),

which is designed to receive the sensor signal (116) and to determine at least one current end-expiratory gas flow (132) based on the profile (114) of the gas flow-dependent measured values (115),
the processing unit (130) being further designed to adjust, depending on a comparison between the determined current end-expiratory gas flow (132) and a lower threshold value (134) and/or an upper threshold value (135), a ratio (136) between inspiratory time (124) and expiratory time (126) for the currently present ventilation mode for ventilating the patient (102),
the duration of the corresponding respiratory cycle remaining substantially constant, **characterized in that**
the processing unit (130) is designed to adjust, depending on the adjustment of the ratio (136) between inspiratory time (124) and expiratory time (126), an increase in a gas pressure of the respiratory gas at the beginning of the inspiratory time (124) and/or a maximum pressure of the respiratory gas during the inspiratory time (124).

2. Ventilator (100) according to claim 1,
   wherein the processing unit (130) is designed to shorten the expiratory time (126) if the current end-expiratory gas flow (132) is below the lower threshold value (134).

3. Ventilator (100) according to claim 1 and claim 2,

   wherein the processing unit (130) provides a time control in which the current end-expiratory gas flow (132) acts as a measured variable of the time control and the inspiratory time (124) acts as a manipulated variable of the time control, and
   wherein the lower threshold value (134) represents a target value of the measured variable.

4. Ventilator (100) according to claim 1 and claim 2,
   wherein the processing unit (130) is designed to extend the expiratory time (126) if the current end-expiratory gas flow (132) is above the upper threshold value (135).

5. Ventilator (100) according to at least one of the preceding claims,
   wherein the lower threshold value (134) and/or the upper threshold value (135) are dependent on the measured profile (114) of the gas flow-dependent measured values (115).

6. Ventilator (100) according to claim 5,
   wherein the lower threshold value (134) and/or the upper threshold value (135) are dependent on a maximum expiratory gas flow (255) determined from the gas flow-dependent measured values (115) during the expiratory time (126) of the provided ventilation.

7. Ventilator (100) according to at least one of the preceding claims,
   wherein the processing unit (130) is further designed to reduce the increase in the gas pressure at the beginning of the inspiratory time (124) and/or to reduce the maximum pressure of the respiratory gas if the inspiratory time (124) is extended.

8. Ventilator (100) according to at least one of the preceding claims,
   wherein the processing unit (130) is further designed to determine, via the profile (114) of the gas flow-dependent measured values (115), a current end-inspiratory gas flow (465) and to adjust, depending on the current end-inspiratory gas flow (465), a ramp duration (450) of the increase in the gas pressure of the respiratory gas at the beginning of the inspiratory time (124).

9. Ventilator (100) according to claim 8,
   wherein the processing unit (130) is designed to extend the ramp duration (450) if the current end-inspiratory gas flow (465) is below a lower inspiratory threshold value (468), and at the same time to shorten a remaining plateau duration (470) of the inspiratory time (124) so that the inspiratory time (124) is independent of the end-inspiratory gas flow (465).

10. Ventilator (100) according to claim 8 and claim 9,

    wherein the processing unit (130) provides a ramp control in which the current end-inspiratory gas flow (465) acts as a measured variable of the ramp control, and the ramp duration (450) acts as a manipulated variable of the ramp control, and
    wherein the lower inspiratory threshold value (468) represents a target value of the measured variable.

**11.** Ventilator (100) according to claim 8 and claim 9, wherein the processing unit (130) is designed to shorten the ramp duration (450) if the current end-inspiratory gas flow (465) is above an upper inspiratory threshold value, and at the same time to extend a remaining plateau duration (470) of the inspiratory time (124) so that the inspiratory time (124) is independent of the end-inspiratory gas flow (465).

**12.** Ventilator (100) according to at least one of the preceding claims, wherein the processing unit (130) is further designed to extend the expiratory time (126) and/or to extend the ramp duration (450) at constant maximum pressure only if, as a result, a mean ventilation pressure over the corresponding respiratory cycle is not below a predetermined lower mean pressure threshold value.

**Revendications**

**1.** Respirateur (100) pour la respiration mécanique d'un patient (102), comportant

- au moins une unité formant capteur (110), laquelle est configurée pour mesurer une courbe (114) de valeurs de mesure dépendant du flux de gaz (115) dans le circuit de respiration (105) du respirateur (100) et pour émettre un signal de capteur (116) correspondant,
- une unité de stockage (120),

dans laquelle est enregistrée une pluralité de paramètres de respiration (122) d'un mode de respiration actuellement présent, dans lequel lesdits paramètres de respiration (122) enregistrés indiquent au moins un temps d'inspiration (124) de la respiration actuellement fournie par le respirateur (100) et un temps d'expiration (126) subséquent pour un cycle respiratoire correspondant,

- une unité de traitement (130),

laquelle est configurée pour recevoir le signal de capteur (116) et pour déterminer, sur la base de la courbe (114) des valeurs de mesure dépendant du flux de gaz (115), au moins un flux de gaz actuel de fin d'expiration (132), dans lequel l'unité de traitement (130) est en outre configurée pour ajuster, en fonction d'une comparaison entre le flux de gaz actuel de fin d'expiration (132) déterminé et une valeur seuil inférieure (134) et/ou une valeur seuil supérieure (135), un rapport (136) entre le temps d'inspiration (124) et le temps d'expiration (126) pour le mode de respiration actuellement présent pour la respiration du patient (102),

dans lequel la durée du cycle respiratoire correspondant reste sensiblement constante,

**caractérisé en ce que**

l'unité de traitement (130) est configurée pour régler, en fonction du réglage du rapport (136) entre le temps d'inspiration (124) et le temps d'expiration (126), une augmentation d'une pression de gaz du gaz respiratoire au début du temps d'inspiration (124) et/ou une pression maximale du gaz respiratoire pendant le temps d'inspiration (124).

**2.** Respirateur (100) selon la revendication 1, dans lequel l'unité de traitement (130) est configurée pour raccourcir le temps d'expiration (126) si le flux de gaz actuel de fin d'expiration (132) est inférieur à la valeur seuil inférieure (134).

**3.** Respirateur (100) selon les revendications 1 et 2,

dans lequel l'unité de traitement (130) fournit une régulation temporelle lors de laquelle le flux de gaz actuel de fin d'expiration (132) fonctionne comme grandeur de mesure de la régulation temporelle et le temps d'inspiration (124) fonctionne comme grandeur de réglage de la régulation temporelle, et dans lequel la valeur seuil inférieure (134) représente une valeur de consigne de la grandeur de mesure.

**4.** Respirateur (100) selon les revendications 1 et 2, dans lequel l'unité de traitement (130) est configurée pour prolonger le temps d'expiration (126) si le flux de gaz actuel de fin d'expiration (132) est supérieur à la valeur seuil supérieure (135).

**5.** Respirateur (100) selon au moins l'une des revendications précédentes, dans lequel la valeur seuil inférieure (134) et/ou la valeur seuil supérieure (135) dépendent de la courbe (114) mesurée des valeurs de mesure dépendant du flux de gaz (115).

**6.** Respirateur (100) selon la revendication 5, dans lequel la valeur seuil inférieure (134) et/ou la valeur seuil supérieure (135) dépendent d'un flux de gaz maximal de fin d'expiration (255) déterminé à partir des valeurs de mesure dépendant du flux de gaz (115) pendant la durée d'expiration (126) de la respiration fournie.

**7.** Respirateur (100) selon au moins l'une des revendications précédentes,
dans lequel l'unité de traitement (130) est en outre configurée pour réduire l'augmentation de la pression de gaz au début du temps d'inspiration (124) et/ou pour réduire la pression maximale du gaz respiratoire si le temps d'inspiration (124) est prolongé.

**8.** Respirateur (100) selon au moins l'une des revendications précédentes,
dans lequel l'unité de traitement (130) est en outre configurée pour déterminer un flux de gaz actuel de fin d'inspiration (465) par l'intermédiaire de la courbe (114) des valeurs de mesure dépendant du flux de gaz (115) et pour régler, en fonction du flux de gaz actuel de fin d'inspiration (465), une durée de rampe (450) de l'augmentation de la pression de gaz du gaz respiratoire au début du temps d'inspiration (124).

**9.** Respirateur (100) selon la revendication 8,
dans lequel l'unité de traitement (130) est configurée pour prolonger la durée de rampe (450) si le flux de gaz actuel de fin d'inspiration (465) est inférieur à une valeur seuil inspiratoire inférieure (468), et pour raccourcir simultanément une durée de plateau résiduelle (470) du temps d'inspiration (124), de sorte que le temps d'inspiration (124) est indépendant du flux de gaz de fin d'inspiration (465).

**10.** Respirateur (100) selon les revendications 8 et 9,

dans lequel l'unité de traitement (130) fournit une régulation de rampe lors de laquelle le flux de gaz actuel de fin d'inspiration (465) fonctionne comme grandeur de mesure de la régulation de rampe et la durée de rampe (450) fonctionne comme grandeur de réglage de la régulation de rampe, et
dans lequel la valeur seuil inspiratoire inférieure (468) représente une valeur de consigne de la grandeur de mesure.

**11.** Respirateur (100) selon les revendications 8 et 9,
dans lequel l'unité de traitement (130) est configurée pour raccourcir la durée de rampe (450) si le flux de gaz actuel de fin d'inspiration (465) est supérieur à une valeur seuil inspiratoire supérieure, et pour prolonger simultanément une durée de plateau résiduelle (470) du temps d'inspiration (124), de sorte que le temps d'inspiration (124) est indépendant du flux de gaz actuel de fin d'inspiration (465).

**12.** Respirateur (100) selon au moins l'une des revendications précédentes,
dans lequel l'unité de traitement (130) est en outre configurée pour ne prolonger le temps d'expiration (126) et/ou ne prolonger la durée de rampe (450) lors d'une pression maximale constante que si, ainsi, une pression respiratoire moyenne au cours du cycle respiratoire correspondant n'est pas inférieure à une valeur seuil de pression moyenne inférieure prédéfinie.

FIG. 1

FIG. 2

FIG. 3

FIG. 4

FIG. 5

FIG. 6

700

710

720

730

740

**FIG. 7**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2016067619 A1
- EP 3769668 A1
- DE 69623400 T2
- EP 0127905 A2
- EP 1961378 A1